# EUROPÄISCHE PATENTSCHRIFT

(11) **EP 3 801 395 B1**
(45) Veröffentlichungstag und Bekanntmachung des Hinweises auf die Patenterteilung: **28.08.2024**
(21) Anmeldenummer: 19711061.2
(22) Anmeldetag: 12.03.2019
(51) Int. Cl.: A61F 2/30, A61B 34/10, A61F 2/00, A61L 31/14

(54) **VERFAHREN UND VORRICHTUNG ZUR HERSTELLUNG EINES IMPLANTATS**
METHOD AND DEVICE FOR PRODUCING AN IMPLANT
PROCÉDÉ ET DISPOSITIF DE FABRICATION D'UN IMPLANT

(30) Priorität: 07.06.2018 DE 102018113580
(43) Veröffentlichungstag der Anmeldung: 14.04.2021
(73) Patentinhaber: Karl, Christoph, 8272 Ermatingen (CH)
(72) Erfinder: RITTER, Zully, 14542 Werder (Havel) (DE); KARL, Christoph, 8272 Ermatingen (CH)
(74) Vertreter: Page, White & Farrer Germany LLP
(86) Internationale Anmeldenummer: PCT/EP2019/056123
(87) Internationale Veröffentlichungsnummer: WO 2019/233641

(56) Entgegenhaltungen:
- EP-A2- 3 266 418
- WO-A1-03/045463
- WO-A1-2015/185219
- WO-A1-2018/108360
- US-A1- 2018 055 643

## Beschreibung

Die vorliegende Erfindung bezieht sich auf ein Verfahren und eine Vorrichtung zur Herstellung eines Implantats und insbesondere auf ein Verfahren und eine Vorrichtung zur Herstellung eines Gelenkimplantats zur Gewebeneubildung am Gelenk wie z.B. ein Gelenkimplantat zur Knorpelneubildung am Knie-, Hüft-, Schulter-, Sprung-, Zehengrund- oder Handgelenk.

Gelenkerkrankungen (Arthrose) gehören zu den zehn häufigsten Erkrankungen weltweit. Arthose ist schmerzhaft und kann ohne Behandlung zur Immobilität der erkrankten Gelenke bis zum totalen Gelenkersatz führen. Ein Totalersatz des erkrankten Gelenks ist hierbei mit hohen Kosten verbunden und wird üblicherweise von den betroffenen Patienten als psychisch belastend empfunden. Revisionen des Gelenkersatzes wirken sich mit weiteren Kosten, Belastungen für den Patienten und häufig Komplikationen aus. Es haben sich daher verschiedene Ansätze zur kurativen Behandlung von Gelenkerkrankungen (Arthrose) und insbesondere zur kurativen Behandlung von Knie- und Hüftgelenks-Arthrose entwickelt, um einen Gelenkersatz zu vermeiden.

Die derzeit existierenden Ansätze zur kurativen Behandlung von Gelenkerkrankungen werden nachfolgend erläutert.

Medikamentöse Therapieoptionen: Die medikamentösen Therapieoptionen beschränken sich auf die symptomatische Anwendung von Antiphlogistika und Analgetika sowie die teils intraartikuläre und teils systemische Behandlung mit Hyaluronsäure, Chondroitinsulfat, Interleukin-1 Rezeptorantagonisten und Glucosaminsulfat. Obwohl hierbei gute Ergebnisse bei der Schmerzreduktion nachgewiesen wurden, konnte ein Fortschreiten der Arthrose bislang jedoch damit nicht verhindert werden.

Operative Therapien: Auch operative Therapien wie lokale Knochen- oder Knorpeltransplantation bzw. autologe Chondrozyten-Transplantation oder -Implantation (ACT oder ACI) konnten sich noch nicht durchsetzen, da hierbei jeweils zwei Operationen notwendig sind (Entnahme und Replantation), was eine lange Entlastung bzw. Ruhigstellung des Gelenks während der Rehabilitation bedeutet und sich somit ungünstig auf die Regenerierung auswirkt. Außerdem wird der noch gesunde Knorpel an der Entnahmestelle geschädigt.

Am weitesten verbreitet sind deshalb operative Therapien wie die sogenannte Pridie-Bohrung, die anterograde/retrograde Anbohrung und die Mikrofrakturierung. Bei diesen operativen Therapien erfolgt kein lokaler Knorpelersatz, sondern es werden z.B. mehrere Bohrungen durch die subchondrale Grenzlamelle durchgeführt. Bei der Bohrung nach Pridie und deren Weiterentwicklung "Mikrofrakturierung" kann im Defektareal ein Einbluten in den Knorpeldefekt erzielt werden, wodurch Fibrozyten, mesenchymale Stammzellen und Chondroblasten aus dem spongiösen Raum in den Knorpeldefekt eingeschwemmt werden. Diese bilden mit Wachstumsfaktoren ein Blutkoagel ("super clot") und differenzieren sich zu artikulärem Knorpel. Klinische Studien zeigten Schmerzverringerung und eine gute Gelenkbeweglichkeit. Ein Problem ist jedoch auch hier eine lange Entlastung bzw. Ruhigstellung des Gelenks, wobei sich ein in der Regel minderwertig regenerativer Faserknorpel entwickelt. Dieser ist aufgrund seiner Struktur für die hohen mechanischen Belastungen insbesondere im Kniegelenk häufig nur unzureichend beschaffen und degeneriert schnell, was erneute Eingriffe erforderlich machen kann.

Aus diesem Grund wurden Carbonstifte als Gelenkimplantate zur Gewebeneubildung am Gelenk entwickelt, welche in die Bohrungen eingesetzt werden und zu einem schnellen Überwachsen führen sollen.

Aus der WO 03/045463 A1 ist ein derartiges Gelenkimplantat zur Gewebeneubildung am Gelenk bekannt, wobei die eingesetzten Stifte aus verdichtetem Kohlenstoff mit einer vorbestimmten Porosität bestehen. Auch bei Verwendung derartiger herkömmlicher Carbonstifte können Fibrozyten und mesenchymale Stammzellen aus dem spongiösen Raum in den Knorpeldefekt eingeschwemmt werden, die ein "super clot" bilden und sich zu artikulärem Knorpel differenzieren.

Das System hat sich jedoch aufgrund zweier wesentlicher Nachteile noch nicht durchgesetzt. Einerseits ist Kohlenstoff als Werkstoff im Knorpel bei Orthopäden nicht akzeptiert, da Mikroabrieb befürchtet wird. Andererseits ist die Oberfläche nicht auf das Ansiedeln von Stammzellen ausgelegt, was sich auch hierbei in der Entwicklung eines minderwertig regenerativen Faserknorpels zeigt.

Aufgrund dieser Limitierungen konnte sich keine der vorstehend genannten Therapien bisher als "Standard of Care" durchsetzen.

Aus der Druckschrift US 2018/0055643 A1 ist eine Vorrichtung und ein Verfahren zur Herstellung eines implantierbaren Gewebegerüsts bekannt, welches mittels eines 3D-Druckverfahrens als Mehrschichtsystem herstellbar ist, wobei zumindest eine der Materialschichten löslich ist und zumindest eine weitere der Materialschichten nicht-löslich ist.

Schließlich ist aus der den Oberbegriff der Patentansprüche 1 und 9 bildenden Druckschrift WO 2015/185219 A1 eine Vorrichtung und ein Verfahren zur Herstellung eines Implantats bekannt, wobei eine natürliche Knochenmikrostruktur eines natürlichen Knochenbereiches erfasst, ein Implantatbereich im natürlichen Knochenbereich markiert, die erfasste Knochenmikrostruktur im markierten Implantatbereich zur Ermittlung von Reproduktionsparametern analysiert, und eine künstliche Mikrostruktur auf der Basis der ermittelten Reproduktionsparameter zur Herstellung des Implantats aufgebaut wird.

Der Erfindung liegt daher die Aufgabe zugrunde, ein Verfahren und eine Vorrichtung zur Herstellung eines Implantats zu schaffen, wobei auf besonders einfache Weise eine chondrozytäre Differenzierung von mesenchymalen Stammzellen begünstigt wird.

Erfindungsgemäß wird diese Aufgabe hinsichtlich des Verfahrens durch die Maßnahmen des Patentanspruchs 1 und hinsichtlich der Vorrichtung durch die Merkmale des Patentanspruchs 9 gelöst.

Durch das Erfassen einer natürlichen Knochenmikrostruktur eines natürlichen Knochenbereiches, dem Markieren eines Implantatbereiches im natürlichen Knochenbereich, dem Analysieren der erfassten Knochenmikrostruktur im markierten Implantatbereich zur Ermittlung von Reproduktionsparametern, und dem Aufbauen einer künstlichen Mikrostruktur auf der Basis der ermittelten Reproduktionsparameter kann ein Implantat hergestellt werden, das eine zum zu behandelnden Knochenbereich sehr ähnliche oder identische Mikrostruktur aufweist. Dadurch kann ein natürlicher Übergang zwischen Implantat und noch gesundem Knochenbereich realisiert werden, wodurch eine natürliche Heilung individuell gefördert wird. Insbesondere durch ein Beschichten der künstlichen Mikrostruktur mit einem hydrophoben chemischen Material kann eine chondrozytäre Differenzierung besonders einfach und wirkungsvoll realisiert werden.

Beispielsweise wird die natürliche Knochenmikrostruktur durch ein hochauflösendes peripheres quantitatives Computertomografie-Verfahren (HR-pQCT) erfasst, welches eine Vielzahl von 2-D-Schnittansichten eines zu untersuchenden Knochenbereichs liefert. Ein 3 dimensionales (3-D) Abbild des zu untersuchenden Knochenbereichs kann dadurch mit besonders hoher Qualität erstellt werden.

Beispielsweise werden beim Analysieren der erfassten Knochenmikrostruktur eine Vielzahl von Scheibenparametern oder eine Vielzahl von Trabekularparametern als Reproduktionsparameter ermittelt, wodurch die Weiterverarbeitung der Daten bzw. die Herstellung des Implantats wesentlich vereinfacht wird.

Vorzugsweise wird das Aufbauen der künstlichen Mikrostruktur durch ein 3D-Druckverfahren realisiert, wodurch sich die Herstellungskosten bei hoher Qualität und Genauigkeit weiter veringern.

Beispielsweise ist die künstliche Mikrostruktur des Implantats eine künstliche Scheibenstapelstruktur oder eine künstliche Trabekularstruktur. Hierdurch werden die Kosten und der Zeitbedarf für die Herstellung weiter verringert.

Vorzugsweise ist das Implantat ein stiftförmiges Gelenkimplantat zur Gewebeneubildung an einem Gelenk, welches eine hydrophobe Oberfläche zur Begünstigung einer chondrozytären Differenzierung von mesenchymalen Stammzellen aufweist. Dadurch kann eine Gewebeneubildung und insbesondere eine Knorpelneubildung am Gelenk sowie die Bildung höherwertigen, belastbaren Knorpels verbessert werden. Auch kann eine langanhaltende und durch den fortlaufenden Transport von mesenchymalen Stammzellen in Richtung des Gelenkspaltes nachhaltige Knorpelregenaration erreicht werden, die weitere Eingriffe am Gelenk hinauszögern oder unnötig werden lassen kann.

Beispielsweise wird als Material bei der Herstellung des Implantats ein Polymer, insbesondere PA, PEK, PEKK, PEEK, UHMWPE oder PCL, ein Metall, insbesondere Ti oder Edelstahl, eine Metall-Legierung, insbesondere Ti64 oder CoCr, eine Magnesium-Legierung, insbesondere Mg-Ca, Mg-Zr oder Mg-Zn, eine Keramik, insbesondere Al₂O₃, ZrO₂ oder Ca₃(PO₄)₂, oder Si₃N₄ oder in vivo resorbierbare Mg-Legierung verwendet, wodurch ein mechanisch hochfestes Implantat mit verbesserten Eigenschaften zur Gebewebeneubildung für die unterschiedlichsten Einsatzbereiche realisiert werden kann.

Beispielsweise kann ein Wachstumsfaktor auf die künstliche Mikrostruktur zur Begünstigung einer chondrozytären Differenzierung von mesenchymalen Stammzellen aufgebracht werden, insbesondere FGF-1, FGF-2, FGF-10 bis FGF-22, SDF-1, IGF-1, PDGF, TGF-β1 und TGF-β3, BMP-2 und BMP-7, OP-1, PRP oder bio-inertes Polyamid, wodurch eine Knorpeldifferenzierung sowie das Anwachsen von Gewebe und insbesondere von Knorpelmaterial am Gelenkimplantat und an der Defektstelle weiter verbessert werden.

Die Vorrichtung zur Herstellung eines Implantats umfasst vorzugsweise eine Erfassungsvorrichtung zum Erfassen einer natürlichen Knochenmikrostruktur eines natürlichen Knochenbereiches, eine Markiervorrichtung zum Markieren eines Implantatbereiches im natürlichen Knochenbereich, eine Analysevor-richtung zum Analysieren der erfassten Knochenmikrostruktur im markierten Implantatbereich und zur Ermittlung von Reproduktionsparametern, und einer Reproduktionsvorrichtung zum Aufbauen einer künstlichen Mikrostruktur auf der Basis der ermittelten Reproduktionsparameter und zur Herstellung des Implantats.

Vorzugsweise ist zumindest eine Übertragungsvorrichtung zum Senden/Empfangen der erfassten Knochenmikrostruktur, des markierten Implantatbereiches und/oder der ermittelten Reproduktionsparameter vorgesehen, wodurch eine besonders effektive und kostengünstige Herstellung von individuellen Implantaten ermöglicht wird.

In den weiteren Unteransprüchen sind weitere vorteilhafte Ausgestaltungen der Erfindung gekennzeichnet.

Die Erfindung wird nachstehend anhand von Ausführungsbeispielen unter Bezugnahme auf die Zeichnung näher beschrieben.

Es zeigen:
Figur 1 eine vereinfachte Schnittansicht eines Oberschenkelknochens mit erfindungsgemäßen Implantaten;
Figuren 2A und 2B vereinfachte perspektivische Ansichten von natürlichen Knochenmikrostrukturen (Trabekularstrukturen) ;
Figuren 3A bis 3F vereinfachte perspektivische Ansichten von künstlichen Trabekularstrukturen;
Figur 4 eine vereinfachte perspektivische Ansicht eines Gelenkimplantats gemäß einem ersten Ausführungsbeispiel der Erfindung;
Figur 5 eine vereinfachte perspektivische Ansicht eines Gelenkimplantats gemäß einem weiteren Ausführungsbeispiel der Erfindung;
Figur 6 eine vereinfachte perspektivische Ansicht eines Gelenkimplantats gemäß einem weiteren Ausführungsbeispiel der Erfindung;
Figur 7 eine schematische Übersicht der verschiedenen Reifungsstufen von mesenchymalen Stammzellen nach Aubin 1998;
Figuren 8A und 8B ein Ausführungsbeispiel zur Herstellung einer hydrophoben chemischen Beschichtung am Beispiel von segmentierten Polyurethanen;
Figur 9A und 9B vergrößerte Ansichten von mit segmentierten PU beschichteten Ti-Substraten zur Veranschaulichung eines jeweiligen Kontaktwinkels; und
Figur 10 eine vereinfachte Blockdarstellung einer Vorrichtung zur Herstellung eines Implantats gemäß einem Ausführungsbeispiel der Erfindung;
Figur 11 eine vereinfachte Draufsicht einer 2-D-Schnittansicht in einem zu untersuchenden Knochenbereich;
Figur 12A bis 12C vereinfachte perspektivische Darstellungen von 2-D-Schnittansichten und einer daraus resultierenden dreidimensionalen markierten natürlichen Knochemikrostruktur in einem zu untersuchenden Knochenbereich; und
Figur 13 ein vereinfachtes Flussdiagramm eines Verfahrens zur Herstellung eines Implantats gemäß einem weiteren Ausführungsbeispiel der Erfindung.

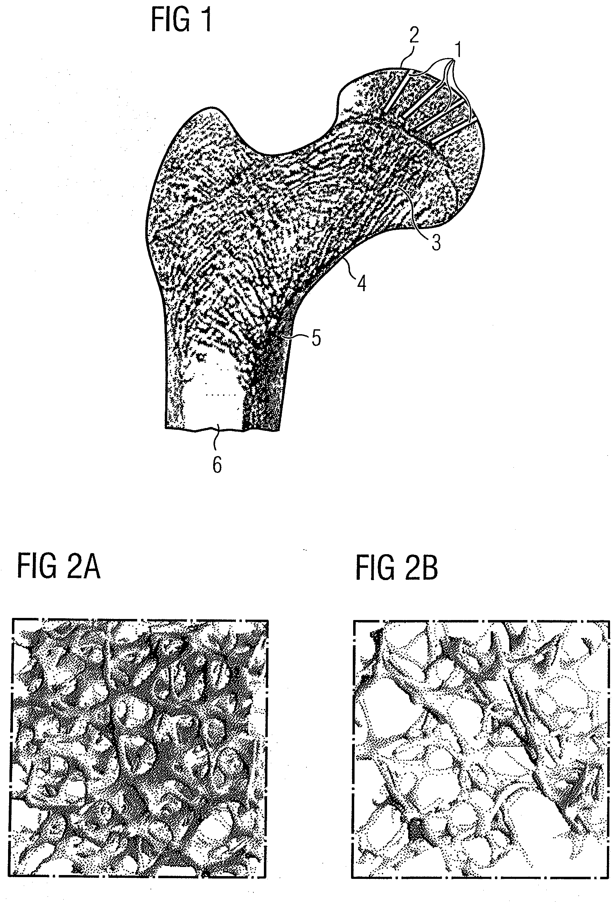

Die Figur 1 zeigt eine vereinfachte Schnittansicht eines oberen Abschnitts eines Oberschenkelknochens zur beispielhaften Veranschaulichung der Verwendung der erfindungsgemäßen Implantate bzw. Gelenkimplantate zur Gewebeneubildung an einem Oberschenkel-/Hüftgelenk. In Figur 1 sind mit Bezugszeichen 1 die erfindungsgemäßen Implantate bzw. Gelenkimplantate dargestellt, welche im Bereich des Gelenks in den Oberschenkelknochen eingebracht werden können. Hierbei kann beispielsweise an beschädigten Knorpelbereichen 2 eine oder eine Vielzahl von Vertiefungen in den Knochen gebohrt, gestanzt oder anderweitig ausgebildet und anschließend ein jeweiliges Gelenkimplantat 1 in die jeweils ausgebildete Vertiefung eingebracht werden. Die jeweilige Vertiefung ist hierbei vorzugsweise derart dimensioniert, dass das eingesetzte Gelenkimplantat 1 bzw. dessen Deckenbereich nicht an der Oberfläche des Knochens bzw. Knorpels 2 herausragt, sondern mit dieser eben abschließt oder vorzugsweise der Deckenbereich des Gelenkimplantats 1 unterhalb der Oberfläche des Knochens bzw. Knorpels 2 liegt. Der aufgrund von Gelenkerkrankung (Arthrose) geschädigte Gelenkknorpel 2 kann mit den erfindungsgemäßen Gelenkimplantaten 1 zumindest teilweise regeneriert werden, da an den Enden bzw. Deckenbereichen der eingebrachten Gelenkimplantate 1 eine Neubildung von Gewebe und insbesondere von Gelenkknorpel erfolgt.

Gemäß Figur 1 weist der Knochen eine den Knochen bedeckende Knochenhaut 4 auf, wobei in den Endbereichen 3 des Knochens eine natürliche Knochenmikrostruktur bzw. natürliche Trabekularstruktur vorliegt, welche als sogenannte Spongiosa bezeichnet wird. Ferner besteht der Knochen in seinem mittleren Bereich aus einer relativ festen Knochenrinde 5 und in seinem Inneren aus einer Knochenmarkhöhle 6.

Durch die Verwendung eines Implantats 1 mit einer künstlichen Mikrostruktur bzw. einer künstlichen Trabekularstruktur, die zur natürlichen Knochenmikrostruktur identisch oder sehr ähnlich ist, können verbesserte Eigenschaften bei der Regeneration und/oder Prävention von geschwächten Knochenbereichen bzw. Geweben erzielt werden, was nachfolgend im Einzelnen erläutert wird.

Die Figuren 2A und 2B zeigen vereinfachte perspektivische Ansichten von natürlichen Knochenmikrostrukturen bzw. Trabekularstrukturen, wie sie beispielsweise im spongiösen Knochenbereich 3 des menschlichen Oberschenkelknochens vorliegen. Hierbei ist gemäß Figur 2A bei einem jungen, gesunden Menschen der spongiöse Knochenbereich 3 mit einer sehr feinen und dichten natürlichen Knochenmikrostruktur bzw. Trabekularstruktur durchsetzt, während gemäß Figur 2B ein älterer und insbesondere ein an z.B. Osteoporose erkrankter Mensch oftmals eine stark veränderte natürliche Knochenmikrostruktur bzw. Trabekularstruktur mit nur wenigen und sehr dünnen Knochenbälkchen (Trabekel) im spongiösen Knochenbereich 3 besitzt.

Beispielsweise kann das Implantat als Gelenkimplantat 1 einen stiftförmigen Körper aufweisen, der eine künstliche Mikrostruktur bzw. Trabekularstruktur aufweist, die zum zu ersetzenden Knochenbereich ähnlich oder identisch ist. Durch die künstliche und zumindest teilweise offene bzw. für Flüssigkeiten durchlässige künstliche Mikrostruktur bzw. Trabekularstruktur des Implantats 1, welche im Wesentlichen der natürlichen Knochenmikrostruktur des zu ersetzenden Knochbereichs entspricht, ist beispielsweise ein schnelles Besiedeln der trabekularen Oberfläche und insbesondere des zum Knorpelbereich zeigenden Teils des stiftförmigen Implantats mit knorpelbildenden Zellen wie z.B. Chondroblasten ermöglicht, was ein signifikant beschleunigtes und zugleich dauerhaftes Überwachsen zur Folge hat und ferner einen hochwertigen regenerativen Knorpel ermöglicht.

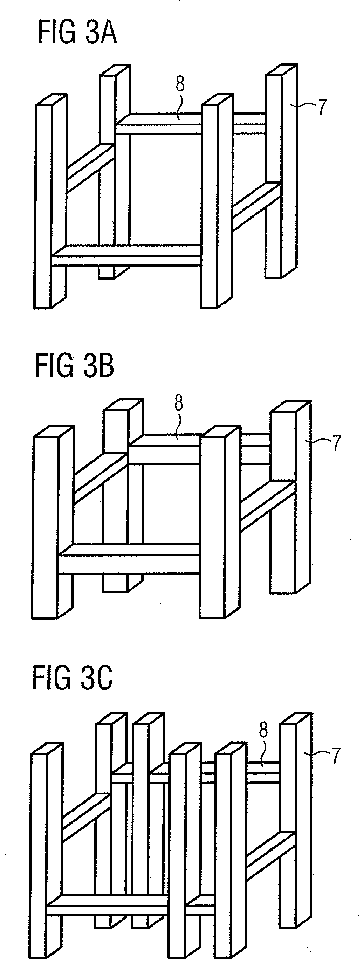

Die Figuren 3A bis 3F zeigen vereinfachte perspektivische Ansichten von künstlichen Trabekularstrukturen. Die künstliche Trabekularstruktur weist hierbei eine Vielzahl von balkenförmigen oder plattenförmigen Elementen (Trabekel) auf, welche miteinander verbunden eine 3-dimensionale Mikroarchitektur ergeben.

Hierbei sollten die durch vorzugsweise 3D-Drucktechnik hergestellten, biomimetischen künstlichen Trabekel bestimmte Parameter nicht unterschreiten bzw. überschreiten.

Nachfolgend werden die maßgeblichen Reproduktionsparameter der erfindungsgemäßen künstlichen Mikrostrukur bzw. Trabekularstruktur näher definiert.

Die sogenannte "Mean Trabecular Thickness" (Tb.Th) definiert die durchschnittliche Trabekeldicke der jeweiligen Trabekel bzw. balkenförmigen Elemente. Da beispielsweise gemäß Figur 3A die jeweiligen Trabekel unterschiedlich ausgeformt sein können, stellt Tb.Th den Durchschnitt der lokalen Dicken aller künstlichen Trabekel dar. Die lokale Dicke ergibt sich z.B. bei rechteckförmigen Trabekeln aus der Trabekel-Diagonale und bei kreisförmigen Trabekeln aus dem Trabekel-Durchmesser. Die Figur 3B zeigt schematisch die Auswirkungen auf die künstliche Mikrostruktur bei einer Zunahme der durchschnittlichen Trabekeldicke Tb.Th. Vorzugsweise liegt die durchschnittliche Trabekeldicke Tb.Th für die künstliche Trabekularstruktur in einem Bereich von 100 bis 500 µm und insbesondere von 150 bis 400 µm.

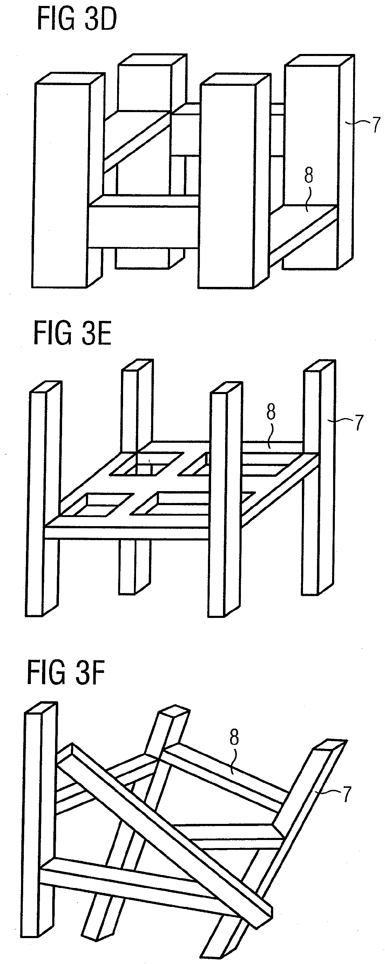

Die sogenannte "Mean Trabecular Separation" (Tb.Sp) definiert den durchschnittlichen Trabekelabstand analog zur durchschnittlichen Trabekeldicke Tb.Th. Eine Abnahme von Tb.Sp kann aus der Änderung verschiedener anderer Parameter resultieren, z.B. Zunahme von Tb.Th (Figur 3B), Zunahme von Tb.N (Figur 3C) oder Abnahme des "Structure Model Index" SMI (Figur 3D). Die Einheit für den durchschnittlichen Trabekelabstand Tb.Sp ist um und liegt für die erfindungsgemäße künstliche Trabekularstruktur vorzugsweise in einem Bereich zwischen 100 µm und 900pm und insbesondere zwischen 200 µm und 600 µm.

Die sogenannte "Trabecular Number" (Tb.N) ist definiert als Umkehrfunktion der mittleren Distanz zwischen den Achsen der Platten und/oder Balken und gibt die Trabekelanzahl pro mm an. Die Figur 3C zeigt beispielsweise eine Zunahme von Tb.N im Vergleich zu Figur 3A. Vorzugsweise liegt die Trabekelanzahl Tb.N für die künstliche Trabekularstruktur in einem Bereich von 1 bis 6 pro mm, insbesondere in einem Bereich von 1,6 bis 5,2 pro mm.

Der sogenannte "Structure Model Index" (SMI) ist ein weiterer beschreibender Parameter der künstlichen Trabekularstruktur, bei dem es sich beispielsweise um ein aus Platten- und Stabähnlichen Elementen aufgebautes Netzwerk handeln kann. In der Realität nimmt das trabekuläre Netzwerk jedoch nicht die eine oder andere Form an, sondern es besteht ein fließender Übergang. Mit zunehmendem Alter geht z.B. ein eher Plattenähnliches Netzwerk in ein eher Stabähnliches über. Ausgehend von dieser Erkenntnis wurde daher der sogenannte "Structure Model Index" (SMI) eingeführt, der es ermöglicht, die Struktur in Bezug auf die Anzahl der Platten und Stäbe zu quantifizieren. Für ein ideales Plattenmodell liegt der SMI bei einem Wert von 0 (d.h. reine Plattenstruktur), für ein ideales Stabmodell bei einem Wert von 3. Der SMI beschreibt also die relative Zusammensetzung der künstlichen Trabekularstruktur aus Platten und Stäben. Figur 3D zeigt schematisch eine Abnahme von SMI. Der SMI ist dimensionslos und liegt für die vorliegende Erfindung beispielsweise bei 0,2 bis 2,0, vorzugsweise bei 0,25 bis 1,8.

Die sogenannte "Connectivity-Density (Conn.D) ist ein Maß für die Vernetzung des trabekulären Geflechts. Konnektivität ist die maximale Anzahl von Verbindungen, die innerhalb des Netzwerkes z.B. durch Mikrofrakturen unterbrochen werden können, ohne das Netz als Ganzes in zwei nicht mehr miteinander verbundene Teile zu unterbrechen. Die Figur 3E zeigt schematisch eine Zunahme der Vernetzungsdichte Conn.D. Vorzugsweise liegt die Vernetzungsdichte Conn.D für die erfindungsgemäße künstliche Trabekularstruktur in einem Bereich von 1/mm³ bis 60/mm³, insbesondere von 1,5/mm3 bis 45/mm3.

Beim geometrischen Grad der Anisotropie (DA) handelt es sich um einen Parameter zur Quantifizierung der räumlichen Asymmetrie. Je höher DA, desto mehr liegt eine Orientierung der künstlichen Trabekularstruktur in einer bestimmten Richtung vor. Die Figur 3F zeigt schematisch eine Abnahme von DA. DA ist wie der Parameter SMI dimensionslos. Ein DA von 0 gibt eine perfekt isotrope, ein DA von 1 eine perfekt anisotrope Struktur an. Ergänzend wird der Grad der Anisoptropie auch durch den sogenannten tDA (alternativer DA) mit Werten von 1, perfekt isotrop, bis unendlich, perfekt anisotrop, angegeben. Der tDA findet bei der Beschreibung der erfindungsgemäßen Struktur hier allerdings keine Anwendung. Vorzugsweise liegt der geometrische Grad der Anisotropie DA für die erfindungsgemäße künstliche Trabekularstruktur in einem Bereich von 0,1 bis 1,0, insbesondere von 0,2 bis 0,8 und weiter bevorzugt bei 0,2 bis 0,6.

Beim sogenannten "Bone Volume Tissue Volume Fraction" (BV/TV) im Spongiosabereich handelt es sich um den volumetrischen Anteil der Trabekel am Gesamtvolumen einer betrachteten Trabekularstruktur. Eine Zunahme von BV/TV kann aus der Änderung verschiedener anderer Parameter resultieren, z.B. Zunahme von Tb.Th (Figur 3B), Zunahme von Tb.N (Figur 3C) oder Abnahme von SMI (Figur 3D). Vorzugsweise liegt BV/TV für die erfindungsgemäße Trabekularstruktur in einem Bereich von 6% bis 70%, stärker bevorzugt von 20% bis 50%.

Schließlich definiert das sogenannte "Marrow Star Volume" (MSV) eine jeweilige Trabekel-Porosität der künstlichen Trabekularstruktur. Genauer gesagt bestimmt die MSV die Größe der Hohlräume in der künstlichen Trabekularstruktur. Der arithmetische Mittelwert mMSV liegt erfindungsgemäß vorzugsweise in einem Bereich von 0,05 mm³ bis 110 mm³, insbesondere zwischen 0,05 mm³ bis 9 mm³ und weiter bevorzugt zwischen 0,05 mm³ bis 5 mm³.

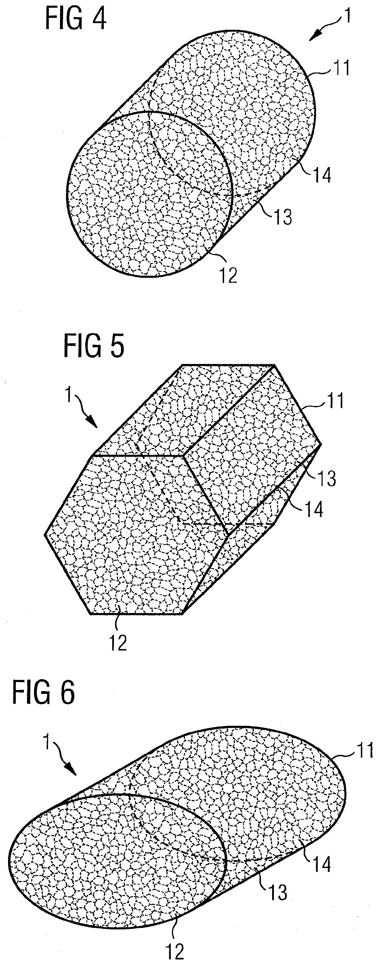

Figur 4 zeigt eine vereinfachte perspektivische Ansicht des Implantats 1 gemäß einem ersten Ausführungsbeispiel der Erfindung. Das Implantat 1 weist hierbei einen stiftförmigen Körper mit einer Makrostrukturierung in Form eines VollZylinders auf. Das Implantat kann als Gelenkimplantat 1 hierbei derart in den Knochen gemäß Figur 1 eingesetzt werden, dass es im Bereich des Knorpels 2 vorzugsweise leicht vertieft im Knochen angeordnet ist. Somit kann ein Deckenbereich 12 des Gelenkimplantats 1 als Wachstumsbereich für das neu auszubildende Gewebe bzw. den neu auszubildenden Gelenkknorpel 2 wirken. Der untere Teil des Gelenkimplantats 1 befindet sich vorzugsweise vollständig im spongiösen Knochenbereich 3.

Gemäß Figur 4 kann die vorstehend beschriebene künstliche Mikrostruktur bzw. Trabekularstruktur 14 im gesamten Volumen des Implantatkörpers ausgebildet werden. Durch die offene und für Körperflüssigkeiten durchlässige künstliche Mikrostruktur bzw. Trabekularstruktur des Gelenkimplantats 1 wird beispielsweise ein schnelles Besiedeln der trabekularen Oberfläche mit Zellen wie Chondroblasten ermöglicht, was ein signifikant beschleunigtes Überwachsen zur Folge hat. Ferner ermöglicht das erfindungsgemäße Gelenkimplantat 1 je nach Struktur und Beschichtung das Wachstum eines regenerativen Faserknorpels bis hin zu einem hochwertigen, hyalinen regenerativen Knorpels insbesondere am Deckenbereich 12.

Vorzugsweise hat der stiftförmige Körper des Gelenkimplantats 1 eine Länge von mindestens 0,6 cm und maximal 1,2 cm für die patellare und Applikation in Kleingelenken wie, z.B. dem Hand- oder Sprunggelenk und mindestens 0,8 cm und maximal 2,2 cm, insbesondere 1,0 cm bis 1,6 cm und weiter bevorzugt 1,25 cm, für die jeweils proximale und distale tibiale und femurale Applikation im Knie- und Hüftgelenk. Dadurch kann ein Optimum an Zugänglichkeit und Einwachsen von mesenchymalen Stammzellen ermöglicht werden. Der stiftförmige Körper des Gelenkimplantats 1 kann ferner einen Durchmesser von mindestens 2 mm und maximal 6 mm, vorzugsweise 3 mm, aufweisen, wodurch ein Optimum an lateraler, der Synovia zugewandten Oberfläche zur Ausbildung von Ersatzknorpelgewebe erzielbar ist.

Durch die individuelle an die natürliche Trabekularstruktur angelehnte Netzstruktur der künstlichen Mikrostruktur bzw. Trabekularstruktur des Implantats 1 kann ein optimales Einwachsen von körpereigenem Gewebe in das Grenzvolumen zwischen Gelenkimplantat 1 und Vertiefung bzw. Bohrkanal, insbesondere in das Innenvolumen des Mantelbereichs 13 des Gelenkimplantats 1 und oberhalb des zur Synovia (Gelenkspalt) zeigenden Endes des Gelenkimplantats 1 ermöglicht werden. Ferner sind die Übergänge (Berührungspunkte) von Implantat 1 auf natürlichen Knochebereich mechanisch optimiert.

Vorzugsweise werden die Gelenkimplantate 1 als mikrostrukturierte Stifte auf Basis von medizinisch zugelassenen, bioinerten und biokompatiblen, 3D-Druck-fähigen Materialien wie beispielsweise nicht-bioresporbierbaren Polymeren, insbesondere Polyamid (PA), Polyetherketone, insbesondere PEK [Polyetherketon], PEKK [Poly(etherketonketon)], PEEK [Polyetheretherketon], Polyethylen (PE), insbesondere UHMWPE [ultra high molecular weight polyethylene], oder z.B. bioresorbierbaren Polymeren, insbesondere PCL [Poly-ε-Caprolacton] ausgebildet.

Alternativ können auch, vorzugsweise 3D-Druck geeignete, Metalle und Metall-Legierungen, insbesondere Titan (Reintitan Grade 1), insbesondere Ti64 (Ti6Al4V), Ti64 ELI und TiCP, Edelstahl, insbesondere 316L, und Cobaltchrom-Legierungen, insbesondere CoCr als Materialien für die Gelenkimplantate 1 und insbesondere für deren künstliche Trabekularstrukturen verwendet werden, einschlißlich resorbierbarer, 3D druckbarer biokompatibeler Metall-legierungen, insbesondere Mg-Legierungen wie Mg-Ca, Mg-Zr und Mg-Zn von hoher Belastbarkeit und Resorptionsrate von 2 mm/Jahr bis zu 3 mm/Jahr. Diese Mg-Legierungen sind vorteilhaft, weil daraus Implantate mit ähhlichen mechanischen Eigenschaften (Dichte (1.8 - 2.1 gm/cm3) und Emodul (30 GPa - 45 GPa) wie bei Knochen hergestellt werden können.

Ferner können auch nicht-bioresporbierbare, vorzugsweise 3D-Druck geeignete, Keramiken, insbesondere AluminiumoxidKeramik [Al2O3], und Zirkondioxid-Keramik [ZrO2], oder bioresporbierbare Keramiken, insbesondere Calciumphosphat-Keramik [Ca3(PO4)2] als Materialien für die Gelenkimplantate 1 verwendet werden.

Vorzugsweise kann auch Si₃N₄ als Material für die künstliche Mikrostruktur bzw. Trabekularstruktur des Implantats bzw. Gelenkimplantats 1 verwendet werden.

Grundsätzlich können auch weitere medizinisch zugelassene, bioinerte und biokompatible, sowie insbesondere 3D-Druckfähige Materialien für die Gelenkimplantate 1 und insbesondere für die künstlichen Trabekularstrukturen 14 gemäß Figuren 3 A bis 3F verwendet werden.

Figur 5 zeigt eine vereinfachte perspektivische Ansicht des Gelenkimplantats 1 gemäß einem weiteren Ausführungsbeispiel der Erfindung, wobei gleiche Bezugszeichen gleiche oder entsprechende Elemente bezeichnen, weshalb nachfolgend auf ihre wiederholte Beschreibung verzichtet wird. Gemäß Figur 5 kann der stiftförmige Körper des Gelenkimplantats 1 auch die Form eines Prismas aufweisen. Durch die Makrostrukturierung, z.B. in Form eines mehreckförmigen Querschnitts, erhält man eine weiter verbesserte Verankerung des Gelenkimplantats 1 im Knochen bzw. spongiösen Knochenbereich 3, wodurch sich die Dauerhaltbarkeit der Gelenkimplantate weiter verbessert.

Figur 6 zeigt eine vereinfachte perspektivische Ansicht des stiftförmigen Körpers des Gelenkimplantats 1 gemäß einem weiteren Ausführungsbeispiel der Erfindung, wobei gleiche Bezugszeichen gleiche oder entsprechende Elemente bezeichnen, weshalb nachfolgend auf ihre wiederholte Beschreibung verzichtet wird. Alternativ kann gemäß Figur 6 als Makrostrukturierung auch ein im Querschnitt ellipsenförmiger Körper als Gelenkimplantat 1 verwendet werden, wobei wiederum eine verbesserte Verankerung und insbesondere eine verringerte rotatorische Beweglichkeit um die Längsachse erreicht wird, jedoch das Auftreten von unerwünschten Bruch-Segmenten an Kanten und Ecken verringert ist. Eine Dauerhaltbarkeit ist dadurch weiter erhöht.

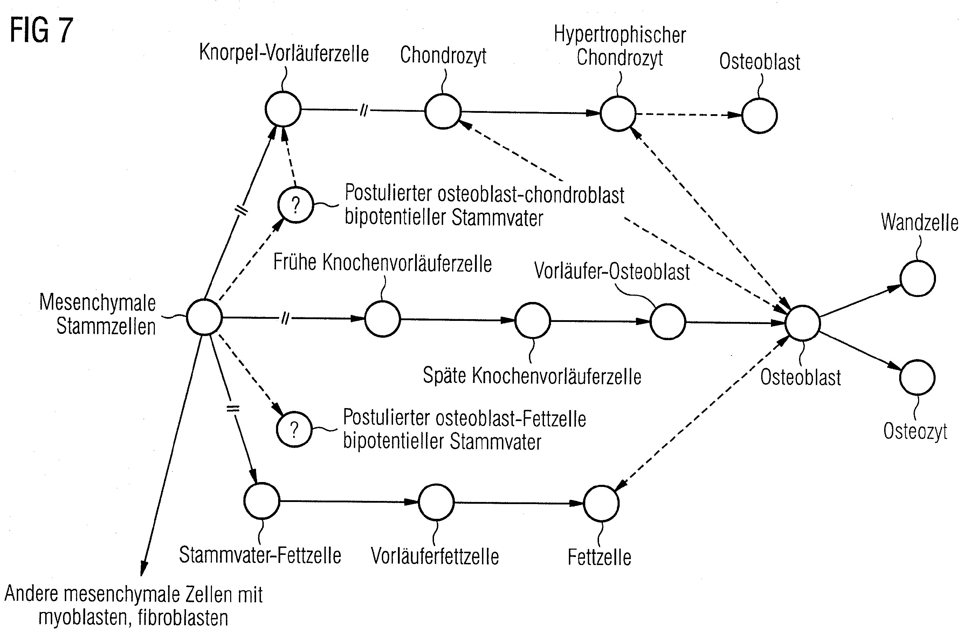

Figur 7 zeigt eine schematische Übersicht der verschiedenen Reifungsstufen von mesenchymalen Stammzellen nach Aubin 1998. Erfindungsgemäß ist es wünschenswert, dass sich mesenchymale Stammzellen (MSC) zumindest im Deckenbereich 12 des stiftförmigen Körpers des Gelenkimplantats 1 zu Chondrozyten differenzieren, um die in diesem Bereich angestrebte Knorpelneubildung zu erzeugen. Andererseits kann im unteren Teil bzw. Bodenbereich 11 des stiftförmigen Körpers des Gelenkimplantats 1 eine Differenzierung der mesenchymalen Stammzellen (MSC) hin zu Osteozyten vorteilhaft sein, um eine Knochenbildung und damit ein optimales Einwachsen des Gelenkimplantats 1 in den spongiösen Knochenbereich 3 zu begünstigen.

Überraschenderweise wurde festgestellt, dass eine derartige Differenzierung von mesenchymalen Stammzellen bereits durch die Erzeugung eines entsprechenden Untergrunds begünstigt werden kann. Genauer gesagt konnte festgestellt werden, dass eine hydrophobe Oberfläche eines Untergrunds eine chondroblastäre und insbesondere chondrozytäre Differenzierung von mesenchymalen Stammzellen und somit eine Knorpelbildung begünstigt, während eine hydrophile Oberfläche eines Substrats bzw. Untergrunds eine osteoblastäre Differenzierung von mesenchymalen Stammzellen und somit eine Knochenbildung begünstigt.

Die Begriffe "Hydrophobie" bzw. "hydrophobe Oberfläche" und "Hydrophilie" bzw. "hydrophile Oberfläche" werden nachfolgend über den sogenannten Kontaktwinkel eines Wassertropfens an einer Oberfläche definiert. Hydrophobe Oberflächen weisen hierbei einen Kontaktwinkel größer oder gleich 90° auf, wobei Kontaktwinkel größer 160° superhydrophobe Oberflächen kennzeichnen. Bekanntester Vertreter für diese superhydrophoben Oberflächen ist die sogenannten "Lotospflanze" Kontaktwinkel von bis zu 170° aufweist. Andererseits sind hydrophile Oberflächen durch einen Kontaktwinkel kleiner 90° gekennzeichnet.

Erfindungsgemäß kann diese Differenzierungs-Eigenschaft der Stammzellen in Abhängigkeit von der Hydrophobie oder Hydrophilie einer Oberfläche dahingehend ausgenutzt werden, dass der stiftförmige Körper des Gelenkimplantats 1 entsprechende hydrophobe Oberflächen aufweist, die eine chondrozytäre Differenzierung der mesenchymalen Stammzellen und somit eine Knorpelausbildung begünstigen.

Hierbei kann sowohl der gesamte stiftförmige Körper eine hydrophobe Oberfläche aufweisen oder aber nur ein Teil des Körpers hydrophobe Oberflächen besitzen. Beispielsweise weist zumindest der Deckenbereich 12 eine hydrophobe Oberfläche auf, um ein Knorpelwachstum an dieser Stelle zu begünstigen. Andererseits kann der stiftförmige Körper in seinem Deckenbereich 12 und oberen Mantelbereich 13 eine hydrophobe Oberfläche aufweisen, während der Bodenbereich 11 und der untere Teil des Mantelbereichs 13 eine hydrophile Oberfläche aufweist. Dadurch kann im oberen (aus dem Knochen herausragenden) Bereich des Gelenkimplantats 1 ein Knorpelwachstum und im unteren (im Knochen befindlichen) Bereich des Gelenkimplantats ein Knochenwachstum begünstigt werden.

Erfindungsgemäß kann die hydrophobe und damit eine die chondrozytäre Differenzierung begünstigende Oberfläche auf verschiedene Art und Weise realisiert werden. Einerseits können chemische Beschichtungen auf den stiftförmigen Körper und insbesondere dessen künstliche Trabekelstrukturen aufgebracht werden, welche die hydrophoben (wasserabweisenden) Eigenschaften verbessern.

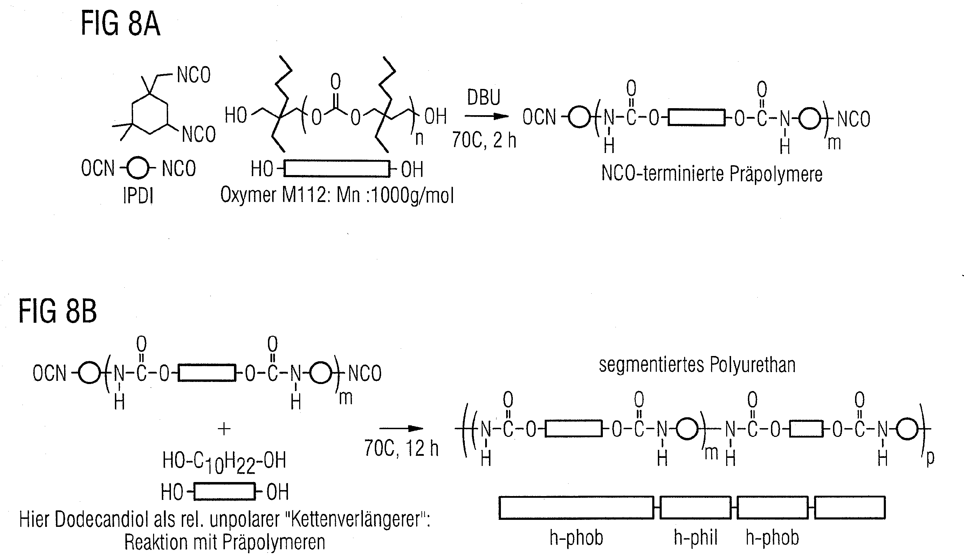

Figuren 8A und 8B zeigen ein Ausführungsbeispiel zur Herstellung einer derartigen hydrophoben Beschichtung am Beispiel von segmentierten Polyurethanen, wie sie auf eine künstliche Mikrostruktur bzw. künstliche Trabekularstruktur des erfindungsgemäßen Gelenkimplantats 1 aufgebracht werden kann.

Gemäß Figur 8A wird zunächst die Herstellung von NCO-terminierten Präpolymeren veranschaulicht, wobei ein stöchiometrischer Überschuss von -NCO vorliegt. Gemäß Figur 8B werden anschließend die NCO-terminierten Präpolymere unter Verwendung von Dodecandiol als unpolaren "Kettenverlängerer" in das gewünschte segmentierte Polyurethan (segmentiertes PU) umgewandelt.

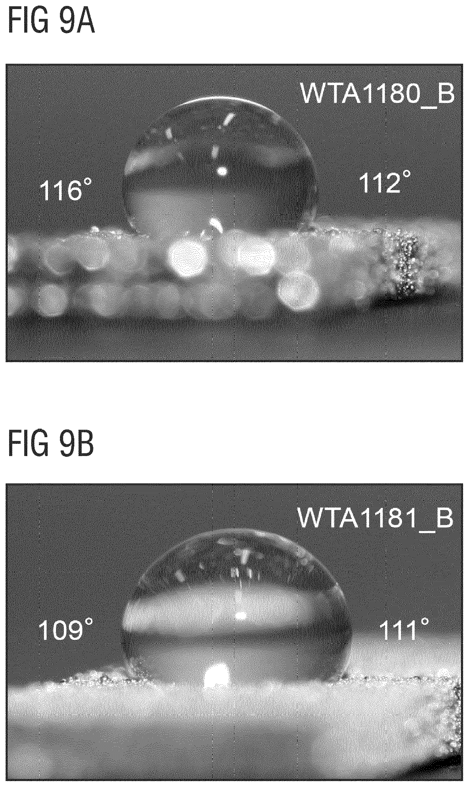

Figur 9A zeigt eine vergrößerte Ansicht eines mit einem derartigen segmentierten PU beschichteten Ti-Substrats. Während ein unbeschichtetes Ti-Substrat (nicht dargestellt) einen Kontaktwinkel von 0° aufweist, besitzt die mit segmentiertem PU beschichtete (10% PU in Toluol) Ti-Oberfläche einen Kontaktwinkel von ca. 112° bis 116°.

Figur 9B zeigt eine vergrößerte Ansicht eines mit einem segmentierten PU beschichteten Ti-Substrats, wobei eine Konzentration des segmentierten PUs bei 2% in Toluol liegt. Die mit einer derartigen segmentierten PU beschichtete Ti-Oberfläche weist nunmehr einen Kontaktwinkel von ca. 109° bis 111° auf.

Für die vorstehend beschriebene hydrophobe PU-Beschichtung wurden folgende Komponenten verwendet:
a) Aliphatische Diisocyanate: Isophoron-Diisocyanat (IPDI), Hexamethylendiisocyanat (HDI) und Dicyclohexylmethandiisocyanat (Hydriertes MDI, HMDI)
b) Polyole: Polycarbonat-Diole (Hydrolysebeständigkeit), wie z.B. Desmophen C2200, Desmophen XP2586, sowie Kohlenwasserstoff-Diole auf der Basis von Naturkautschuk und hydriertem Naturkautschuk
c) Kettenverlängerer: aliphatische Diole wie Hexandiol, Decandiol und eventiuell längere Diole wegen der Hydrophobie

Ferner kann als hydrophobe chemische Beschichtung auch ein Polyelektrolyt-Komplex auf PU-Basis verwendet werden. Hierbei werden die gleichen Komponenten wie oben verwendet, wobei zusätzlich sulfonierte Diole oder ammoniumgruppenhaltige Diole als Kettenverlängerer verwendet werden, um ionische Gruppen für die Bildung der Elektrolytkomplexe einzuführen.

Die Komplexbildung erfolgt dann nach Beschichtung (vorzugsweise Tauchbeschichtung) durch Eintauchen in eine verdünnte Lösung mit einem Tensid (kationisch oder anionisch, je nach dem, welche ionischen Gruppen im Polymer vorhanden sind). Die ionischen Wechselwirkungen zwischen dem Polyelektrolyten und dem Tensid führen zu einer festen Bindung, vor allem, wenn das Tensid hydrophob ist und daher ohnehin in wässriger Umgebung keine Tendenz hat, in Lösung zu gehen.

Ferner kann als hydrophobe chemische Beschichtung auch ein Polyelektrolyt-Komplex auf Acrylat-Basis verwendet werden, wobei eine erste Schicht aus Polyelektrolyten wie Polyacrylsäure bzw. acryl- oder methacrylsäurehaltigen Copolymeren, evtl. auch mit einigen Phosphorsäuregruppen (Comonomer Vinylphosphonsäure) zur Haftung auf der Oberfläche aufgebracht wird und anschließend eine zweite Schicht wie oben aufgebracht wird (Beschichtung aus einer Tensid-Lösung, abgestimmt auf die ionischen Gruppen des Polyelektrolyten).

Vorzugsweise werden folgende drei Typen von hydrophoben Beschichtungsmaterialien verwendet:
Polyurethan vernetzt
Polyurethan unvernetzt
Polyelektrolyt-Komplexe
welche nachfolgende Charakteristika aufweisen:
Polyurethan vernetzt:
Polyurethane unterscheiden sich von den meisten anderen Polymeren und Kunststoffen dadurch, daß sie durch ein "Baukastensystem" aus vielen unterschiedlichen Komponenten (Diisocyanaten, Polyisocyanaten, Polyolen, Kettenverlängerern, Weichsegmenten etc.) zusammengesetzt werden. Dabei erfolgt der eigentliche Aufbau (chemische Synthese der Polymermoleküle) typischerweise erst während der Verarbeitung, so daß der Anwender bzw. Hersteller von Bauteilen auf der Basis von Polyurethanen die endgültigen Eigenschaften ganz nach seinen Anforderungen zusammenstellen kann. Nahezu alle anderen Kunststoffe werden dagegen mit festen Eigenschaftsprofilen vom Rohstoffhersteller (Chemische Industrie) hergestellt und ausgeliefert, so daß der Anwender bzw. Hersteller von Bauteilen nur verhältnismäßig geringen Einfluß auf das Eigenschaftsprofil hat. Daher stellen Polyurethane eine sehr gute Ausgangsbasis für Spezialentwicklungen wie der Beschichtung des erfindungsgemäßen Implantatkörpers 1 dar.

Polyurethane werden seit langem als biokompatible Werkstoffe eingesetzt, beispielsweise als inerte, nicht-abbaubare Beschichtung von Herzschrittmachern, oder auch als biokompatible, abbaubare Trägermaterialien (Scaffolds) für Tissue Engineering bzw. Regenerative Medizin. Die Eigenschaften (z.B. Hydrophobie/Hydrophilie, Abbaubarkeit/Langzeitstabilität, Festigkeit, Steifigkeit, Porosität etc.) werden dabei durch die Kombination der Komponenten nach Bedarf eingestellt.

Vernetzte Polyurethane werden in verdünnter Lösung in Gegenwart des zu beschichtenden Substrats hergestellt. Dabei können die Komponenten so gewählt werden, daß während der Vernetzung gleichzeitig auch eine chemische Anbindung an die Oberfläche des zu beschichtenden Substrats erfolgt. Diese Materialien haften oft ohne Haftvermittler oder ähnliche Zwischenschichten hervorragend, besonders auf hydrophilen Oberflächen. Dabei können die Komponenten der Polyurethane so gewählt werden, daß die entstehenden Schichten selbst hydrophob sind.

Als Komponenten sind für die Biokompatibilität aliphatische Di- und Polyisocyanate geeignet, für die Langzeitstabilität Weichsegmente und Polyole auf Polycarbonat-, Silicon- oder Polybutadien-Basis, und für die Hydrophobie langekttige Diole, eventuell ebenfalls auf Silicon- oder Polybutadien-Basis als Kettenverlängerer.

Der Hauptnachteil ist die problematische Kontrolle der Schichtdicke bei der Beschichtung. Die Konzentration ist der einzige unabhängige Parameter, dessen Variation die Schichtdicke beeinflussen kann. Zwar wirkt sich auch die Zusammensetzung und die Reaktionszeit auf die Schichtdicke aus, allerdings beeinflusst die Zusammensetzung auch alle anderen Eigenschaften, und die Reaktionszeit kann nicht beliebig festgesetzt werden, denn für die Biokompatibilität ist die vollständige Umsetzung der Isocyanatgruppen erforderlich, so daß die Beschichtungszeit nicht beliebig verkürzt werden kann.

Polyurethane, unvernetzt:
Unvernetzte Polyurethane werden getrennt vom Beschichtungsvorgang hergestellt und anschließend aus einer verdünnten Lösung in einem Tauchvorgang aufgebracht. Die Einstellung der Eigenschaften bietet die gleichen Möglichkeiten wie bei den vernetzten Polyurethanen, da fast alle Komponenten in beiden Fällen eingesetzt werden können.

Der Vorteil der unvernetzten Polyurethane liegt darin, daß Synthese und Beschichtung getrennt voneinander ablaufen, so daß bessere Möglichkeiten zur Steuerung der Schichtdicke bestehen. Die Konzentration, die Einwirkzeit beim Tauchvorgang, und vor allem die Anzahl der Tauchvorgänge (mit jeweils Trocknung dazwischen) bestimmen die Dicke der aufgebrachten Schicht.

Der Nachteil liegt darin, daß eine chemische Anbindung der Schicht an das Substrat entweder eine Haftvermittler-Schicht erfordert, oder spezielle Komponenten im Polyurethan, die mit der Oberfläche reagieren können. Unter Umständen ist die Haftung dieser Schichten daher weniger dauerhaft, oder der Beschichtungsvorgang ist aufwändiger, da vor der eigentlichen Beschichtung zunächst eine Haftvermittlerschicht aufgebracht werden muß. Da dies aber voraussichtlich ebenfalls als einfache Tauchbeschichtung möglich ist, ist der zusätzliche Aufwand begrenzt.

Polyelektrolytkomplexe:
Polyelektrolytkomplexe machen sich elektrostatische Wechselwirkungen zwischen positiv und negativ geladenen Ionen und Oberflächen zu nutze. Jedes Material weist in Wasser eine bestimmte Oberflächenladung auf (Zeta-Potential), die - je nach der chemischen Struktur - positiv oder negativ ist. Diese Oberflächenladung besitzen auch neutrale Partikel bzw. Oberflächen. Polyelektrolyte, deren Ladungen entlang der Polymerkette dieser Oberflächenladung entgegengesetzt geladen sind, haften auf der Oberfläche sehr fest. Im Allgemeinen können sie nicht mehr entfernt werden, da jede Polymerkette je nach Kettenlänge mit dutzenden oder hunderten Gruppen gleichzeitig haftet und so selbst dann in Position gehalten wird, wenn einige dieser Gruppen durch äußere Einwirkungen abgelöst werden. Auf diese Polyelektrolyte können dann entweder gegensinnig geladene Polyelektrolyte abgeschieden werden, so daß eine auf molekularer Basis genaue Einstellung der Schichtdicke durch abwechselnde Anscheidung (layer-by-layer-Technik) möglich ist. Oder es werden niedermolekulare Ionen, z.B. Tenside bzw. Seifen abgeschieden, deren eines Ende eine dem Polyelektrolyten gegensinnige Ladung trägt um die Haftung sicherzustellen, und deren anderes Ende hydrophob ist. Im Idealfall können so Schichten hergestellt werden, die nach Außen hin eine dichte Schicht an z.B. Methylgruppen aufweisen, womit nahezu die Oberflächenspannung von Fluorpolymeren (PTFE, Teflon) erreicht werden kann.

Die Vorteile dieser Materialien liegen in der üblicherweise hervorragenden Haftung in wässrigen oder nicht-wässrigen Systemen, in der exakten Steuerbarkeit der Schichtdicke, und in der relativ gut kontrollierbaren, sehr ausgeprägten Hydrophobie.

Nachteilig ist die Abscheidung in nahezu monomolekularen Schichten, die bei größeren Schichtdicken ein große Zahl von Tauchvorgängen in abwechselnden Polyelektrolytbädern erfordert. Da allerdings keine Trockenschritte dazwischen erforderlich sind, ist der Aufwand vertretbar.

Ferner können als Ausgangsmaterial für den stiftförmigen Körper des Gelenkimplantats 1 derartige 3D-druckfähige Materialien verwendet werden, die bereits per se (also ohne zusätzliche Mikro- und/oder Nanostrukturierung und/oder chemische Beschichtung eine hydrophobe Oberfläche aufweisen. Beispielsweise zeigt die unbehandelte Oberfläche eines Zirkondioxid-Keramik-Substrats bereits hydrophobe Eigenschaften.

Darüber hinaus können die künstlichen Mikrostrukturen bzw. Trabekularstrukturen 14 zum Zweck der verbesserten Knorpelzelldifferenzierung und des verbesserten Anwachsens von Knorpelmaterial eine zusätzliche Wachstumsbeschichtung bzw. einen Wachstumsfaktor aufweisen. Vorzugsweise kann die künstliche Trabekularstruktur 14 mit spezifischem, humanem und humanhomologem Wachstumsfaktor FGF [Fibroblast Growth Factor] insbesondere FGF-1, FGF-2 und FGF-10 bis FGF-22 und dort insbesondere FGF-18 beschichtet werden. Alternativ kann die künstliche Trabekularstruktur 14 mit spezifischem, humanem und human-homologem Wachstumsfaktor SDF [Stromal Cell-Derived Factor] insbesondere SDF-1 beschichtet werden. Ferner kann spezifischer, humaner und human-homologer Wachstumsfaktor IGF-1 [Insulin-like Growth Factor 1], humanes PDGF [Platelet-Derived Growth Factor], spezifischer, humaner und human-homologer Wachstumsfaktor TGF-β1 und TGF-β3 [Transforming Growth Factors beta 1 und beta 3], oder spezifischer, humaner und human-homologer BMP-2 und BMP-7 [Bone Morphogenetic Protein-2 und Protein-7] auf die künstliche Trabekularstruktur 14 aufgebracht werden. Weitere Möglichkeiten der Beschichtung umfassen: spezifisches, humanes und human-homologes OP-1 [Osteogenic Protein-1], humanes PRP [Platelet-rich Plasma]sowie speziell für Beschichtungen geeignetes bio-inertes Polyamid. Selbstverständlich sind auch Kombinationen der vorstehend beschriebenen Beschichtungen möglich. Vorzugsweise kann der Wachstumsfaktor als letzte Schicht aufgebracht werden.

Erfindungsgemäß kann durch entsprechende Auswahl von geeigneten bioinerten und biokompatiblen Materialien mit idealer Anpassung der geometrischen und chemisch/biochemischen Oberflächenstruktur (künstliche Trabekularstruktur) die Differenzierung von mesenchymalen Stammzellen zu Chondroblasten oder Osteoblasten gezielt gesteuert werden. Dadurch kann insbesondere die Knorpelstruktur auf der der Synovia zugewandten Seite (Deckenbereich 12) der Gelenkimplantate 1 durch die vorstehend beschriebenen hydrophoben Beschichtungen sowie mit den die Knorpelbildung anregenden Wachstumsfaktoren verbessert werden. Weiterhin kann auf der der Synovia abgewandten Seite (Bodenbereich 11) der Gelenkimplantate durch hydrophile Oberflächenstrukturen und Beschichtungen die Knochenbildung sowie die Knochenstruktur im spongiösen Knochenbereich verbessert werden. Dadurch erhält man nahezu physiologische Adäquatheit, da die Gelenkanatomie und die natürliche Knochenstabilität nicht oder nur unbedeutend beeinflusst werden wie z.B. bei Implantation einer Endoprothese. Die Verträglichkeit und Wirksamkeit der kurativen Therapie mittels vorstehend beschriebener Gelenkimplantate ist dadurch wesentlich verbessert.

Durch die Kombination der vorstehend beschriebenen biokompatiblen, bio-inerten, 3D-druckfähigen Materialien, der spezifisch geeigneten bio-medizinischen Geometrien (Krümmung, Nanostruktur, Mikrostruktur und Makrostruktur) und der wachstumsfördernden Beschichtungen erhält man ein neuartiges Gelenkimplantat, welches die Quantität und Qualität und damit die Belastbarkeit und Beständigkeit von Ersatzknorpelgewebe weiter optimieren kann und wesentlich zur kurativen Behandlung von Gelenkerkrankungen (Arthrose) beitragen kann.

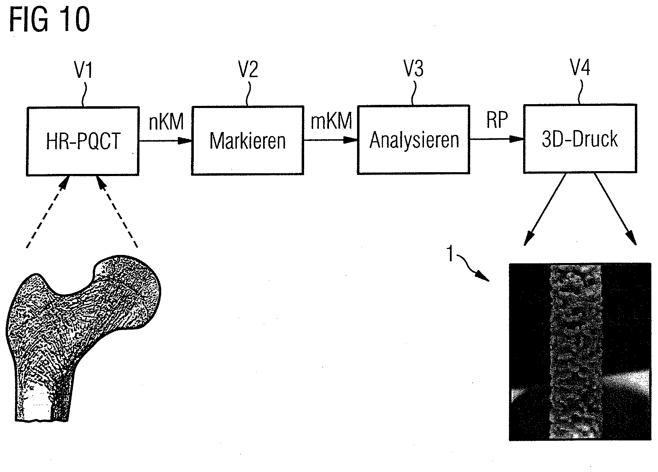

Die Figur 10 zeigt eine vereinfachte Blockdarstellung einer Vorrichtung zur Herstellung eines Implantats 1 gemäß einem bevorzugten Ausführungsbeispiel der Erfindung.

Gemäß Figur 10 erfasst eine Erfassungsvorrichtung V1 eine natürliche Knochenmikrostruktur eines natürlichen Knochenbereichs, wie beispielsweise den in Figur 1 dargestellten Teil eines Oberschenkelknochens. Die Erfassungsvorrichtung V1 kann beispielsweise einen hochauflösenden peripher quantitativen Computertomografen (HR-pQCT, high resolution pheripheral quantitative computed tomography) aufweisen. Alternativ können jedoch auch andere Bildgebungsvorrichtungen verwendet werden, die eine ausreichend genaue dreidimensionale Wiedergabe einer jeweiligen Knochenmikrostruktur ermöglichen.

Beispielsweise erzeugt die Erfassungsrichtung V1 eine Vielzahl von zweidimensionalen (2-D) Schnittansichten im zu untersuchenden Knochenbereich. Die bei Versuchen eingesezte Erfassungsvorrichtung XtremeCT^{®} weist z.B. eine Auflösung von 82 µm auf, wodurch sich bei 110 Schnittansichten eine Tiefe des untersuchten Knochenbereichs von 9,02 mm ergibt, was für das vorliegende Gelenkimplantat ausreichend ist.

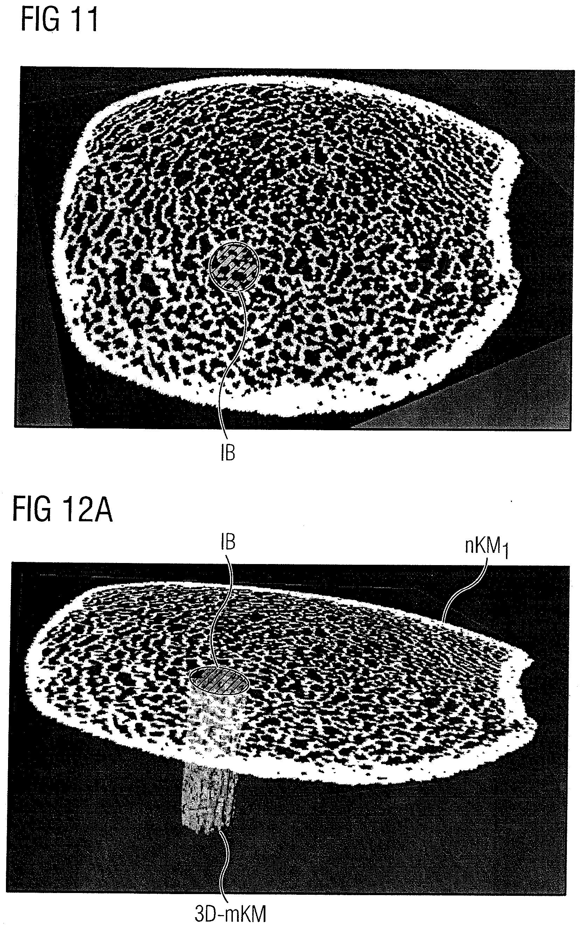

Figur 11 zeigt eine vereinfachte Darstellung einer derartigen 2-D-Schnittansicht in einem zu untersuchenden Knochenbereich.

Mittels der in Figur 10 dargestellten Markiervorrichtung V2 kann nunmehr von einem Nutzer (z.B. behandelnder Arzt) ein Implantatbereich IB festgelegt werden. Hierbei wird im Wesentlichen eine Abtastorientierung (Richtung in der die Schnittansichten erzeugt werden), eine Länge (Anzahl von Schnittansichten + Auflösung (bzw. Abstand der Schnittansichten)) sowie eine zweidimensionale Makrostruktur (Kreis, Mehreck, Ellipse) für einen vorbestimmten Knochenbereich ausgewählt und markiert.

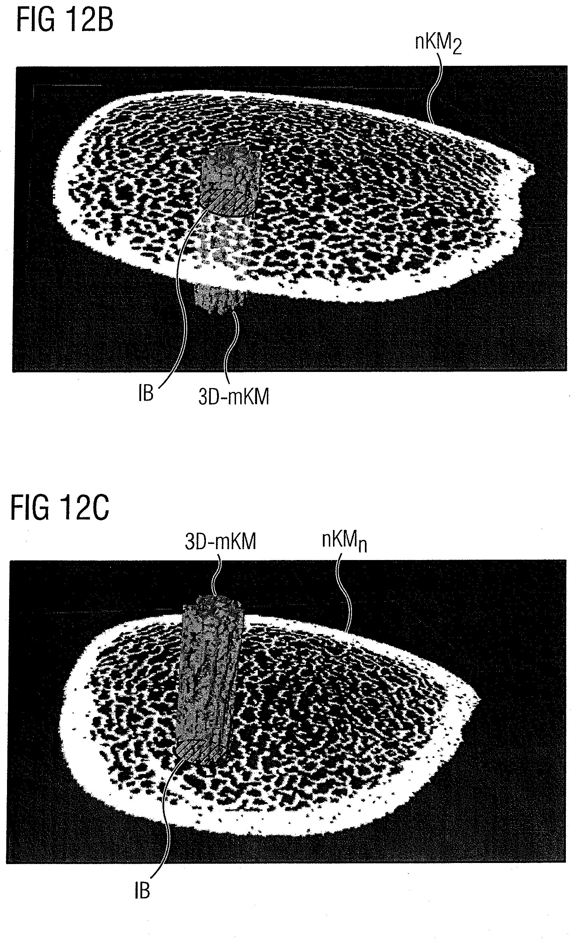

Wie in den Figuren 12A bis 12C vereinfacht dargestellt ist, erhält man auf der Grundlage der Vielzahl von zweidimensionalen Schnittansichten nKM₁ bis nKMₙ der natürlichen Knochenmikrostruktur nKM und der mittels der Markiervorrichtung V2 durchgeführten Markierung des geplanten Implantatbereichs IB ein exaktes Abbild 3D-mKM der markierten natürlichen Knochenmikrostruktur für das gewünschte Implantat bzw. die erfasste Knochenmikrostruktur für den markierten Implantatbereich IB.

Mittels der in Figur 10 dargestellten Analysevorrichtung V3 kann weiterhin eine Analyse dieses Abbilds 3D-mKM bzw. der erfassten Knochenmikrostruktur im markierten Implantatbereich IB zur Ermittlung von Reproduktionsparametern RP durchgeführt werden.

Beispielsweise können eine Vielzahl von Scheibenparametern ermittelt werden, welche im Wesentlichen auf den Daten der zweidimensionalen Schnittansichten und einer Dicke der Scheiben, z.B. Auflösung der Erfassungsvorrichtung V1, basieren. In diesem Fall vereinfacht die Analysevorrichtung V3 jede erfasste zweidimensionale Schnittansicht nKM₁ bis nKMₙ zu einer Scheibe mit vorbestimmter Dicke (z.B. Auflösung), wobei die zweidimensionale Struktur für die gesamte Scheibendicke (d.h. von oben bis unten) gleich ist.

Alternativ kann die erfasste Knochenmikrostruktur 3D-mKM im markierten Implantatbereich bzw. können die Vielzahl von erfassten zweidimensionalen Schnittansichten nKM₁ bis nKMₙ auch in eine Vielzahl von Trabekularparametern als Reproduktionsparameter umgewandelt werden. Im Gegensatz zu den vorstehend beschriebenen Scheibenparametern, die eine Abstufung von Scheibe zu Scheibe erzeugen, wird hierbei im Wesentlichen eine Linearisierung für die nicht erfassten Zwischenräume zwischen den erfassten zweidimensionalen Schnittansichten nKM₁ bis nKMₙ durchgeführt um die Trabekularparameter als Reproduktionsparameter RP zur erzeugen. Ein derartiges Analyseverfahren kann die Genauigkeit bei der Wiedergabe der natürlichen Knochenmikrostruktur weiter verbessern. Weiterhin kann dadurch eine Datenmenge bzw. eine für eine Datenübertragung relevante Datenrate erheblich reduziert werden, da die Trabekularparameter im Wesentlichen Vektordaten darstellen.

Abschließend wird in der Reproduktionsvorrichtung V4 gemäß Figur 10 ein Aufbau einer künstlichen Mikrostruktur bzw. Trabekularstruktur 14 auf der Basis der ermittelten Reproduktionsparameter und zur Herstellung des gewünschten Implantats I durchgeführt. Beispielsweise kann die Reproduktionsvorrichtung V4 einen 3-D-Drucker aufweisen, wodurch eine patientenspezifische Individualisierung von jeweiligen Implantaten kostengünstig ermöglicht wird.

Der Aufbau der künstlichen Mikrostruktur bzw. Trabekularstruktur 14 des Implantats I ist somit sehr ähnlich oder nahezu identisch mit dem markierten natürlichen Knochenbereich eines jeweiligen Patienten, wodurch sich individuell optimale Eigenschaften für die natürliche Heilung und für die mechanische Anpassung des Implantats 1 an die natürliche Knochenmikrostruktur (Knochenkontinuität) eines jeweiligen Patienten ergeben.

Bei Verwendung von Scheibenparametern als Reproduktionsparameter ergibt sich für die künstliche Mikrostruktur des Implantats 1 folglich eine künstliche Scheibenstapelstruktur. Andererseits ergibt sich bei Verwendung von Trabekularparametern als Reproduktionsparameter für die künstlichen Mikrostruktur 14 des Implantats eine künstliche Trabekularstruktur.

Ferner kann zumindest eine Übertragungsvorrichtung (nicht dargestellt) zum Senden/Empfangen der erfassten natürlichen Knochenmikrostruktur nKM, der markierten natürlichen Knochenmikrostruktur mKM und/oder der ermittelten Reproduktionsparameter RP vorgesehen werden, wodurch individuelle Implantate besonders effizient und kostengünstig hergestellt werden können. Die Übertragungsvorrichtung kann die Daten z.B. über gesicherte Kanäle im Internet insbesondere zu zentralen Analysezentren und/oder Reproduktionszentren übertragen.

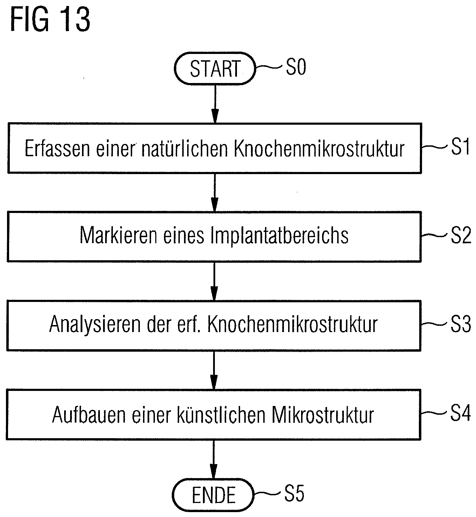

Figur 13 zeigt ein vereinfachtes Flussdiagramm eines Verfahrens zur Herstellung eines Implantats.

Gemäß Figur 13 wird nach einem Start in Schritt S0 zunächst ein Erfassen einer natürlichen Knochenmikrostruktur, beispielsweise durch erzeugen einer Vielzahl von Schnittansichten nKM₁ bis nKMₙ, durchgeführt. In einem Schritt S2 wird anschließend ein Markieren eines Implantatbereiches IB im natürlichen Knochenbereich zum Erzeugen einer markierten natürlichen Knochenmikrostruktur mKM (Makrostruktur) durchgeführt. Weiterhin wird in einem Schritt S3 ein Analysieren der markierten natürlichen Knochenmikrostruktur mKM bzw. der erfassten Knochenmikrostruktur im markierten Implantatbereich IB durchgeführt, um die Reproduktionsparameter RP zu ermitteln. Abschließend wird auf der Grundlage der ermittelten Reproduktionsparameter RP in einem Schritt S4 ein Aufbau einer künstlichen Mikrostruktur zur Herstellung des Implantats 1 durchgeführt. Mit Schritt S5 endet das Verfahren.

Die Figur 13 zeigt folglich ein Flussdiagramm für ein Verfahren und ein Computer-Programm-Produkt gemäß der vorliegenden Erfindung, wobei der erfindungsgemäße Schritt des Beschichten der künstlichen Mikrostruktur mit einem hydrophoben chemischen Material, wobei das hydrophobe chemische Material ein segmentiertes Polyurethan oder Polyelektrolyt oder ein hydrophob funktionalisiertes Chitosan oder Chitosan-Derivat aufweist, in Figur 13 nicht gezeigt ist. Es sei darauf hingewiesen, dass jeder Block oder Schritt des Flussdiagramms und jeweilige Kombinationen von Blöcken im Flussdiagramm durch Computer-Programm-Befehle implementiert sein können. Diese Computer-Programm-Befehle können auf einen Computer oder ein anderes programmierbares Gerät geladen werden, um eine Vorrichtung zu erzeugen, wobei die im Computer oder einem anderen programmierbaren Gerät ausgeführten Befehle Mittel zum Implementieren der Funktionsweisen erzeugen, wie sie in den Schritten des Flussdiagramms dargestellt sind. Diese Computer-Programm-Befehle können ebenfalls in einem digitalen Speichermedium, wie einem geeigneten zentralen (Cloud) oder dezentralen Massenspeicher wie beispielsweise einer CD/DVD, externe Festplatte oder USB gespeichert sein, die einen Computer oder ein anderes programmierbares Gerät zur Realisierung einer bestimmten Funktionalität anweist. Darüber hinaus können die Computer-Programm-Befehle bzw. der Programm-Code in beispielsweise einem Telekommunikationsnetzwerk heruntergeladen werden, um Betriebsschritte hervorzurufen, die auf einem Computer oder einem anderen programmierbaren Gerät ausgeführt werden, um einen computer-implementierten Prozess zu erzeugen, der die Durchführung der Verfahrensschritte gemäß Figur 13 ermöglicht.

Die Erfindung umfasst daher ferner ein digitales Speichermedium mit elektronisch auslesbaren Steuersignalen, die so mit einem Computer-System zusammenwirken können, dass sie die Verfahrensschritte gemäß Figur 13, und den oben erwähnten, in Figur 13 nicht dargestellten Verfahrensschritt, ausführen können. Ferner bezieht sich die Erfindung auf ein Computer-Programm-Produkt mit auf einem maschinenlesbaren Träger gespeicherten Programm-Code zur Durchführung der Verfahrensschritte nach Figur 13, und des oben erwähnten, in Figur 13 nicht dargestellten Verfahrensschritts, wenn das Programm auf einem Rechner abläuft. Im Übrigen betrifft die vorliegende Erfindung ein Computer-Programm mit Programm-Code zur Durchführung von Verfahrensschritten nach Figur 13, und des oben erwähnten, in Figur 13 nicht dargestellten Verfahrensschritts, wenn das Programm auf einem Computer abläuft.

Die Erfindung wurde vorstehend anhand von bevorzugten Ausführungsbeispielen beschrieben. Sie ist jedoch nicht darauf beschränkt und umfasst insbesondere auch Einzel-Kombinationen der vorstehend beschriebenen Ausführungsbeispiele. Insbesondere kann auch die Vorstufe der chondrozytären Differenzierung von mesenchymalen Stammzellen, nämlich die chondroblastäre Differenzierung von mesenchymalen Stammzellen, durch die hydrophobe Oberfläche begünstigt werden.

Obwohl die Erfindung vorstehend im Zusammenhang mit der Verwendung in menschlichen Hüft- und Kniegelenken beschrieben wurde, ist sie nicht darauf beschränkt und kann insbesondere auch mit menschlichen Klein- sowie Kleinstgelenken (z.B. Fuß- sowie Finger-Gelenken) und Gelenken von Tieren verwendet werden.

### Bezugszeichenliste

- 1: Implantat
- 2: Gelenkknorpel
- 3: spongiöser Knochenbereich
- 4: Knochenhaut
- 5: Knochenrinde
- 6: Knochenmarkhöhle
- 7, 8: Trabekel
- 11: Bodenbereich
- 12: Deckenbereich
- 13: Mantelbereich
- 14: künstliche Mikrostruktur
- V1: Erfassungsvorrichtung
- V2: Markiervorrichtung
- V3: Analysevorrichtung
- V4: Reproduktionsvorrichtung
- IB: Implantatbereich
- nKMₓ: natürliche Knochenmikrostruktur
- mKM, 3D-mKM: markierte Knochenmikrostruktur
- RP: Reproduktionsparameter
- S0 bis S5: Verfahrensschritte

## Patentansprüche

1. Verfahren zur Herstellung eines Implantats mit den Schritten:
a) Erfassen (S1) einer natürlichen Knochenmikrostruktur (nKM) eines natürlichen Knochenbereiches;
b) Markieren (S2) eines Implantatbereiches (IB) im natürlichen Knochenbereich;
b) Analysieren (S3) der erfassten Knochenmikrostruktur (3D-mKM) im markierten Implantatbereich zur Ermittlung von Reproduktionsparametern; und
c) Aufbauen (S4) einer künstlichen Mikrostruktur (14) auf der Basis der ermittelten Reproduktionsparameter zur Herstellung des Implantats (1),
**gekennzeichnet durch** den weiteren Schritt:
d) Beschichten der künstlichen Mikrostruktur (14) mit einem hydrophoben chemischen Material, wobei das hydrophobe chemische Material ein segmentiertes Polyurethan oder Polyelektrolyt oder ein hydrophob funktionalisiertes Chitosan oder Chitosan-Derivat aufweist.

2. Verfahren nach Patentanspruch 1, wobei beim Erfassen der natürlichen Knochenmikrostruktur eine Vielzahl von 2-D-Schnittansichten (nKM₁ bis nKMₙ) durch ein hochauflösendes peripheres quantitatives Computertomografie-Verfahren (HR-pQCT) erzeugt wird.

3. Verfahren nach Patentanspruch 1 oder 2, wobei beim Analysieren eine Vielzahl von Scheibenparametern zum Definieren einer zweidimensionalen Schnittansicht der erfassten Knochenmikrostruktur (3D-mKM) oder eine Vielzahl von Trabekularparametern zum Definieren einer Trabekularstruktur der erfassten Knochenmikrostruktur (3D-mKM) als Reproduktionsparameter (RP) ermittelt werden.

4. Verfahren nach einem der Patentansprüche 1 bis 3, wobei das Aufbauen der künstlichen Mikrostruktur (14) durch ein 3D-Druckverfahren realisiert wird.

5. Verfahren nach einem der Patentansprüche 3 oder 4, wobei die künstliche Mikrostruktur (14) eine den Scheibenparametern entsprechende künstliche Scheibenstapelstruktur oder eine den Trabekularparametern entsprechende künstliche Trabekularstruktur darstellt.

6. Verfahren nach einem der Patentansprüche 1 bis 5, wobei das Implantat (1) ein stiftförmiges Gelenkimplantat zur Gewebeneubildung an einem Gelenk ist, welches eine hydrophobe Oberfläche zur Begünstigung einer chondrozytären Differenzierung von mesenchymalen Stammzellen aufweist.

7. Verfahren nach einem der Patentansprüche 1 bis 6, wobei als Material bei der Herstellung des Implantats ein Polymer, insbesondere PA, PEK, PEKK, PEEK, UHMWPE oder PCL,
ein Metall, insbesondere Ti oder Edelstahl,
eine Metall-Legierung, insbesondere Ti64 oder CoCr,
eine Magnesium-Legierung, insbesondere Mg-Ca, Mg-Zr oder Mg-Zn,
eine Keramik, insbesondere Al₂O₃, ZrO₂ oder Ca₃(PO₄)₂, oder Si₃N₄ verwendet wird.

8. Verfahren nach einem der Patentansprüche 1 bis 7, mit dem weiteren Schritt:
e) Aufbringen eines Wachstumsfaktors auf die künstliche Mikrostruktur (14) zur Begünstigung einer chondrozytären Differenzierung von mesenchymalen Stammzellen, insbesondere FGF-1, FGF-2, FGF-10 bis FGF-22, SDF-1, IGF-1, PDGF, TGF-β1 und TGF-β3, BMP-2 und BMP-7, OP-1, PRP oder bio-inertes Polyamid.

9. Vorrichtung zur Herstellung eines Implantats mit:
einer Erfassungsvorrichtung (V1) zum Erfassen einer natürlichen Knochenmikrostruktur (nKM) eines natürlichen Knochenbereiches;
einer Markiervorrichtung (V2) zum Markieren eines Implantatbereiches (IB) im natürlichen Knochenbereich;
einer Analysevorrichtung (V3) zum Analysieren der im markierten Implantatbereich erfassten Knochenmikrostruktur (mKM) und zur Ermittlung von Reproduktionsparametern (RP);
einer Reproduktionsvorrichtung (V4) zum Aufbauen einer künstlichen Mikrostruktur (14) auf der Basis der ermittelten Reproduktionsparameter (RP) und zur Herstellung des Implantats (1), **gekennzeichnet durch**
eine Beschichtungsvorrichtung zum Beschichten der künstlichen Mikrostruktur (14) mit einem hydrophoben chemischen Material, wobei das hydrophobe chemische Material ein segmentiertes Polyurethan oder Polyelektrolyt oder ein hydrophob funktionalisiertes Chitosan oder Chitosan-Derivat aufweist.

10. Vorrichtung nach Patentanspruch 9, ferner umfassend:
zumindest eine Übertragungsvorrichtung zum Senden/Empfangen der erfassten Knochenmikrostruktur (nKM), der markierten Knochenmikrostruktur (mKM) und/oder der ermittelten Reproduktionsparameter (RP).

11. Vorrichtung nach Patentanspruch 9 oder 10, wobei die Erfassungsvorrichtung (V1) einen hochauflösenden peripher quantitativen Computertomografen (HR-pQCT) aufweist.

12. Vorrichtung nach einem der Patentansprüche 9 bis 11, wobei die Reproduktionsvorrichtung (V4) einen 3-D-Drucker aufweist.

13. Digitales Speichermedium mit elektronisch auslesbaren Steuersignalen, die so mit einem Computersystem, einer Reproduktionsvorrichtung und einer Beschichtungsvorrichtung zusammenwirken können, dass ein Verfahren nach einem der Patentansprüche 1 bis 8 ausgeführt wird.

14. Computer-Programm-Produkt mit auf einem maschinenlesbaren Träger gespeichertem Programmcode zur Durchführung des Verfahrens nach einem der Patentansprüche 1 bis 8, wenn das Programm auf einem Computersystem mit einer Reproduktionsvorrichtung und einer Beschichtungsvorrichtung abläuft.

15. Computer-Programm mit Programmcode zur Durchführung des Verfahrens nach einem der Patentansprüche 1 bis 8, wenn das Programm auf einem Computersystem mit einer Reproduktionsvorrichtung und einer Beschichtungsvorrichtung abläuft.

## Claims

1. Method for producing an implant, comprising the steps of:
a) capturing (S1) a natural bone microstructure (nKM) of a natural bone area;
b) marking (S2) an implant area (IB) in the natural bone area;
b) analysing (S3) the captured bone microstructure (3D-mKM) in the marked implant area to ascertain reproduction parameters; and
c) constructing (S4) an artificial microstructure (14) on the basis of the ascertained reproduction parameters to produce the implant (1),
**characterized by** the further step of:
d) coating the artificial microstructure (14) with a hydrophobic chemical material, wherein the hydrophobic chemical material comprises a segmented polyurethane or polyelectrolyte or a hydrophobically functionalized chitosan or chitosan derivative.

2. Method according to Claim 1, wherein a multiplicity of 2D sectional views (nKM₁ to nKMₙ) is generated by a high-resolution peripheral quantitative computed tomography method (HR-pQCT) when capturing the natural bone microstructure.

3. Method according to Claim 1 or 2, wherein a multiplicity of disc parameters for defining a two-dimensional sectional view of the captured bone microstructure (3D-mKM) or a multiplicity of trabecular parameters for defining a trabecular structure of the captured bone microstructure (3D-mKM) are ascertained as reproduction parameters (RP) when analysing.

4. Method according to one of Claims 1 to 3, wherein constructing the artificial microstructure (14) is realized by means of a 3D printing method.

5. Method according to either of Claims 3 and 4, wherein the artificial microstructure (14) is an artificial stacked-disc structure corresponding to the disc parameters or an artificial trabecular structure corresponding to the trabecular parameters.

6. Method according to one of Claims 1 to 5, wherein the implant (1) is a rod-shaped joint implant for new tissue formation at a joint, which has a hydrophobic surface for facilitating chondrocyte differentiation of mesenchymal stem cells.

7. Method according to one of Claims 1 to 6, wherein the material used in the production of the implant is a polymer, in particular PA, PEK, PEKK, PEEK, UHMWPE or PCL,
a metal, in particular Ti or stainless steel,
a metal alloy, in particular Ti64 or CoCr,
a magnesium alloy, in particular Mg-Ca, Mg-Zr or Mg-Zn,
a ceramic, in particular Al₂O₃, ZrO₂ or Ca₃(PO₄)₂, or Si₃N₄.

8. Method according to one of Claims 1 to 7, having the further step of:
e) applying a growth factor to the artificial microstructure (14) for facilitating chondrocyte differentiation of mesenchymal stem cells, in particular FGF-1, FGF-2, FGF-10 to FGF-22, SDF-1, IGF-1, PDGF, TGF-β1 and TGF-β3, BMP-2 and BMP-7, OP-1, PRP or bioinert polyamide.

9. Device for producing an implant, comprising:
a capture device (V1) for capturing a natural bone microstructure (nKM) of a natural bone area;
a marking device (V2) for marking an implant area (IB) in the natural bone area;
an analysis device (V3) for analysing the bone microstructure (mKM) captured in the marked implant area and for ascertaining reproduction parameters (RP);
a reproduction device (V4) for constructing an artificial microstructure (14) on the basis of the ascertained reproduction parameters (RP) and for producing the implant (1), **characterized by**
a coating device for coating the artificial microstructure (14) with a hydrophobic chemical material, wherein the hydrophobic chemical material comprises a segmented polyurethane or polyelectrolyte or a hydrophobically functionalized chitosan or chitosan derivative.

10. Device according to Claim 9, further comprising:
at least one transmission device for sending/receiving the captured bone microstructure (nKM), the marked bone microstructure (mKM) and/or the ascertained reproduction parameters (RP).

11. Device according to Claim 9 or 10, wherein the capture device (V1) comprises a high-resolution peripheral quantitative computed tomograph (HR-pQCT).

12. Device according to one of Claims 9 to 11, wherein the reproduction device (V4) comprises a 3D printer.

13. Digital storage medium with electronically readable control signals which can interact with a computer system, a reproduction device and a coating device such that a method according to one of Claims 1 to 8 is executed.

14. Computer program product with program code stored on a machine-readable medium for carrying out the method according to one of Claims 1 to 8, when the program runs on a computer system with a reproduction device and a coating device.

15. Computer program with program code for carrying out the method according to one of Claims 1 to 8, when the program runs on a computer system with a reproduction device and a coating device.

## Revendications

1. Procédé de fabrication d'un implant, comprenant ces étapes consistant à :
a) détecter (S1) une microstructure osseuse naturelle (nKM) d'une zone osseuse naturelle ;
b) marquer (S2) une zone d'implant (IB) dans la zone osseuse naturelle ;
b) analyser (S3) la microstructure osseuse détectée (3D-mKM) dans la zone d'implant marquée afin de déterminer des paramètres de reproduction ; et
c) construire (S4) une microstructure artificielle (14) sur la base des paramètres de reproduction déterminés afin de fabriquer l'implant (1),
**caractérisé par** cette étape supplémentaire consistant à :
d) revêtir la microstructure artificielle (14) d'un matériau chimique hydrophobe, dans lequel le matériau chimique hydrophobe comprend un polyuréthane, ou un polyélectrolyte, segmenté, ou bien un chitosane, ou un dérivé de chitosane, fonctionnalisé de manière hydrophobe.

2. Procédé selon la revendication 1, dans lequel, lors de la détection de la microstructure osseuse naturelle, de multiples vues en coupe 2D (nKM₁ à nKMₙ) sont générées par un procédé de tomodensitométrie quantitative périphérique à haute résolution (HR-pQCT).

3. Procédé selon la revendication 1 ou 2, dans lequel, lors de l'analyse, de multiples paramètres de disque destinés à définir une vue en coupe bidimensionnelle de la microstructure osseuse détectée (3D-mKM) ou de multiples paramètres trabéculaires destinés à définir une structure trabéculaire de la microstructure détectée (3D-mKM) sont déterminés en tant que paramètres de reproduction (RP).

4. Procédé selon l'une quelconque des revendications 1 à 3, dans lequel la construction de la microstructure artificielle (14) est réalisée par un procédé d'impression 3D.

5. Procédé selon la revendication 3 ou 4, dans lequel la microstructure artificielle (14) représente une structure artificielle à empilement de disques correspondant aux paramètres de disque ou une structure trabéculaire artificielle correspondant aux paramètres trabéculaires.

6. Procédé selon l'une quelconque des revendications 1 à 5, dans lequel l'implant (1) est un implant articulaire en forme de broche qui est destiné à la régénération tissulaire au niveau d'une articulation et qui présente une surface hydrophobe qui est destinée à favoriser une différenciation chondrocytaire de cellules souches mésenchymateuses.

7. Procédé selon l'une quelconque des revendications 1 à 6, dans lequel le matériau utilisé dans la fabrication de l'implant est : un polymère, en particulier PA, PEK, PEKK, PEEK, UHMWPE ou PCL ; un métal, en particulier Ti ou un acier inoxydable ; un alliage métallique, en particulier Ti64 ou CoCr ; un alliage de magnésium, en particulier Mg-Ca, Mg-Zr, ou Mg-Zn ; une céramique, en particulier Al₂O₃, ZrO₂ ou Ca₃(PO₄)₂, ou Si₃N₄.

8. Procédé selon l'une quelconque des revendications 1 à 7, comprenant cette étape supplémentaire consistant à :
e) appliquer un facteur de croissance sur la microstructure artificielle (14), lequel est destiné à favoriser une différenciation chondrocytaire de cellules souches mésenchymateuses, en particulier FGF-1, FGF-2, FGF-10 jusqu'à FGF-22, SDF-1, IGF-1, PDGF, TGF-β1 et TGF-β3, BMP-2 et BMP-7, OP-1, PRP ou un polyamide bio-inerte.

9. Dispositif de fabrication d'un implant, comprenant :
un dispositif de détection (V1) qui est destiné à détecter une microstructure osseuse naturelle (nKM) d'une zone osseuse naturelle ;
un dispositif de marquage (V2) qui est destiné à marquer une zone d'implant (IB) dans la zone osseuse naturelle ;
un dispositif d'analyse (V3) qui est destiné à analyser la microstructure osseuse (mKM) détectée dans la zone d'implant marquée, et à déterminer des paramètres de reproduction (RP)
un dispositif de reproduction (V4) qui est destiné à construire une microstructure artificielle (14) sur la base des paramètres de reproduction (RP) déterminés, et à fabriquer l'implant (1),
**caractérisé par** :
un dispositif de revêtement qui est destiné à revêtir la microstructure artificielle (14) d'un matériau chimique hydrophobe, dans lequel le matériau chimique hydrophobe comprend un polyuréthane, ou un polyélectrolyte, segmenté, ou bien un chitosane, ou un dérivé de chitosane, fonctionnalisé de manière hydrophobique.

10. Dispositif selon la revendication 9, comprenant en outre :
au moins un dispositif de transmission qui est destiné à envoyer/recevoir la microstructure osseuse détectée (nKM), la microstructure osseuse marquée (mKM), et/ou les paramètres de reproduction (RP) déterminés.

11. Dispositif selon la revendication 9 ou 10, dans lequel le dispositif de détection (V1) comprend un appareil de tomodensitométrie quantitative périphérique à haute résolution (HR-pQCT).

12. Dispositif selon l'une quelconque des revendications 9 à 11, dans lequel le dispositif de reproduction (V4) comprend une imprimante 3D.

13. Support de stockage numérique, lequel est doté de signaux de commande lisibles électroniquement qui peuvent interagir avec un système informatique, un dispositif de reproduction et un dispositif de revêtement de sorte à mettre en oeuvre un procédé selon l'une quelconque des revendications 1 à 8.

14. Produit de programme informatique, lequel est doté d'un code de programme qui est stocké sur un support lisible par une machine et qui est destiné à mettre en oeuvre le procédé selon l'une quelconque des revendications 1 à 8 lorsque le programme est exécuté sur un système informatique doté d'un dispositif de reproduction et d'un dispositif de revêtement.

15. Programme informatique, lequel est doté d'un code de programme qui est destiné à mettre en oeuvre le procédé selon l'une quelconque des revendications 1 à 8 lorsque le programme est exécuté sur un système informatique doté d'un dispositif de reproduction et d'un dispositif de revêtement.
